Europäisches Patentamt

(19) European Patent Office

Office européen des brevets

(11) Publication number: **0 077 815**
**B1**

# EUROPEAN PATENT SPECIFICATION

(45) Date of publication of the patent specification:
22.08.84

(51) Int. Cl.³: **C 07 C 45/49,** C 07 C 45/50

(21) Application number: **82901679.9**

(22) Date of filing: **19.04.82**

(86) International application number:
**PCT/US 82/00483**

(87) International publication number:
**WO 82/03856 (11.11.82 Gazette 82/27)**

(54) **HYDROFORMYLATION PROCESS.**

(30) Priority: **01.05.81 US 259399**

(43) Date of publication of application:
**04.05.83 Bulletin 83/18**

(45) Publication of the grant of the patent:
**22.08.84 Bulletin 84/34**

(84) Designated Contracting States:
**DE FR GB**

(56) References cited:
**FR - A - 2 370 022**
**US - A - 2 880 241**
**US - A - 3 463 741**
**US - A - 3 520 937**
**US - A - 3 527 809**
**US - A - 3 579 575**
**US - A - 3 755 393**
**US - A - 3 920 754**
**US - A - 3 932 523**
**US - A - 4 113 754**
**US - A - 4 221 743**
**US - A - 4 225 458**

(73) Proprietor: **EASTMAN KODAK COMPANY, 343 State Street, Rochester New York 14650 (US)**

(72) Inventor: **COOPER, James Lee, 508 Noel Street, Longview, TX 75601 (US)**

(74) Representative: **Pepper, John Herbert et al, KODAK LIMITED Patent Department P.O. Box 114 190 High Holborn, London WC1V 7EA (GB)**

ACTORUM AG

## Description

This invention relates to a process for the hydroformylation of an olefin in the presence of a rhodium-containing catalyst. More specifically, this invention relates to stabilizing the rhodium against forming deposits (sometimes referred to as plating out) during distillation of a liquid composition formed in such process. This is accomplished by contacting the liquid composition with an oxygen-containing gas and then subjecting such composition to controlled distillation.

The hydroformylation of olefins in liquid solvent with hydrogen and carbon monoxide in the presence of rhodium-containing catalysts to form useful oxygenated products such as aldehydes is well known. See, for example, U.S. Patent No. 2880241, issued March 31, 1959. In both batch and continuous operation, the more volatile components of the reaction mixture are often separated in the form of a gas by distillation. Unfortunately, with many of the rhodium-containing catalysts employed in such hydroformylation processes, distillation of the reaction mixture cause rhodium metal to deposit on the distillation equipment, e.g., in the distillation column or in the base heater for the distillation column. Since such rhodium deposits cannot be recovered or regenerated in any practical way, costly rhodium is lost. In order to provide economically attractive hydroformylation processes of the type described, it is necessary to avoid, at least in part, this loss of rhodium.

This invention provides a hydroformylation process in which the formation of rhodium deposits is inhibited. This process comprises (1) forming an aldehyde product in a liquid composition by hydroformylating an olefin in liquid solvent with hydrogen and carbon monoxide in the presence of a rhodium-containing catalyst which is capable of forming rhodium deposits on distillation apparatus during distillation of the liquid composition and (2) separating aldehyde product in the form of a gas from the liquid composition by distillation, and is characterized in that the liquid composition (*a*) is contacted with an oxygen-containing gas to thereby form a rhodium salt of a carboxylic acid and then (*b*) is subjected to the distillation at a temperature up to 120°C.

An important feature of the process of this invention is that it provides a means for obtaining a higher proportion of valuable branched-chain aldehyde products relative to unbranched-chain aldehyde products in comparison to conventional oxo processes that employ catalysts such as phosphine-modified cobalt. For example, with butyraldehyde oxo products, the lowest molar ratio of normal to isobutyraldehyde obtainable with such commercial cobalt catalysts is in the order of 1.6 to 1.8. A lower ratio is desirable since isobutyraldehyde is a valuable precursor for such materials as isobutyric acid, neopentyl glycol which is a well-known component of coatings and 2,2,4-trimethyl-1-3-pentanediol monoisobutyrate which is a coalescing aid for paints. Another important branched aldehyde is isovaleraldehyde, a precursor to isovaleric acid which is a valuable nutrient additive for cattle feed. The process of this invention provides high proportions of branched aldehyde products, e.g., in the case of butyraldehyde, the ratio of normal to isobutyraldehyde generally ranges from 0.9 to 1.5.

As previously indicated herein, treatment of the liquid composition prior to distillation with an oxygen-containing gas forms a rhodium salt of a carboxylic acid, e.g., rhodium isobutyrate, that does not form the undesirable rhodium deposits during subsequent distillation. Also, such rhodium carboxylates rapidly form active catalyst species upon being returned to the hydroformylation reaction. It is important that the distillation temperature be maintained below 120°C, preferably below 110°C, to insure that essentially complete catalytic activity will be restored by the oxygen treatment.

Rhodium-containing catalysts that are capable of forming the undesired rhodium deposits described previously are well known to those skilled in the art. Such catalysts are described, for example, in U.S. Patent No. 2880241, issued March 31, 1959, referred to previously herein. As indicated in this patent, the catalysts are present in a soluble form during the hydroformylation reaction. Elemental rhodium, inorganic or organic rhodium salts are examples of the substances which will form a soluble rhodium compound under the reaction conditions of the present process. The rhodium may be in the form of a carbonyl which would include any rhodium carbonyl, hydrocarbonyl, mixtures and complex compounds thereof. Accordingly, the catalyst can be added as metallic rhodium, a rhodium oxide, salt or any rhodium-containing composition of matter which can be maintained in the form of a soluble rhodium compound under said reaction conditions. More specifically rhodium dioxide, sesquioxide, halide nitrate, sulfate, sulfite and salts of organic acids, such as isobutyric, oleic or stearic, can be employed.

Rhodium carbonyls are usable and may be preformed in any known manner to form, e.g., a solid crystalline material or a solution with an organic solvent, e.g., hexane, aldehyde, olefin or alcohol, and either may be used in the reaction mixture as the catalyst. Rhodium carbonyl may be made by any of the various methods known in the art among which direct union of carbon monoxide with metallic rhodium or a rhodium salt at high pressures is effective. Rhodium carbonyl takes several forms, e.g., $Rh_2(CO)_8$, $(Rh(CO)_3)_n$, $(Rh_4(CO)_{11})_n$ and $Rh(CO_4)_4H$. The various forms of rhodium carbonyl as well as mixtures thereof are suitable catalytic materials for the present reaction. Rhodium-containing hydroformylation catalysts in which the rhodium is associated with ligands such as alkyl or arylphosphines, phosphites, arsines and stibines are also well known, as illustrated, e.g., by U.S. Patent No. 3527809, issued September 8, 1970. However, unlike the rhodium-containing catalysts described in this and the preceding paragraph (which are free of Group VA elements), the aforementioned ligand-containing catalysts do not normally form significant rhodium deposits upon distillation equipement. Accordingly, there usually is no advantage in applying the oxygen and distillation treatments described herein to liquid compositions containing such ligand-containing catalysts in the absence of catalysts which normally form the undesirable rhodium deposits.

Some specific parameters of the process of this invention include: the unbranched (normal) to branched-chain (iso) aldehydes are generally produced in the ratio of 1.2 or less, the olefins are preferably $\alpha$-olefins having up to 20 carbon atoms, the hydroformylation reaction temperatures are from 20 to 300°C, preferably from 120 to 180°C, and the pressures are from 204 to 69,052 kPa (approximately 15 to 10,000 psig), with from 6,996 to 34,577 kPa (approximately 1,000 to 5,000 psig) being preferred, and from 10,443 to 20,787 kPa (approximately 1,500 to 3,000 psig) being most preferred. In carrying out the present process in a continuous manner, one may use any of the conventional equipment used in the oxo process, as is well known to those skilled in the art.

In operation, the synthesis gas (a mixture of hydrogen and carbon monoxide) is conveniently introduced into the hydroformylation reaction unit in a continuous manner by means, for example, of a primary compressor with the molar ratio of hydrogen to carbon monoxide being selected according to the particular olefin being hydroformylated and the reaction conditions present, as is well known in the art. Generally, the molar ratio of hydrogen to carbon monoxide in the reaction unit will be at least 0.5, usually in the range of from 0.5 to 10, with from 1 to 2 being preferred. The synthesis gas preferably is present in a molar excess (total moles of $H_2$ + CO) with respect to the olefin, and the molar ratio typically varies from 1 to 10, preferably from above 1 to 2.

The olefin is fed to the reaction unit by means of suitable pumps capable of operating under substantial pressures and the feed rates of the olefin and synthesis gas are selected to maintain the molar ratios of these reactants in the reactor unit. Useful olefins include $\alpha$-olefins containing from 2 to 20 carbon atoms and preferably from 2 to 10 carbon atoms. Illustrative of such $\alpha$-olefins are ethylene, propylene, 1-butene, 2-methyl-1-butene, 1-pentene, 1-hexene, 1-heptene, 1-octene, 2-ethyl-1-hexene, 1-dodecene and 1-octadecene. Also useful in the present process are internal olefins such as butene-2 and cyclic olefins such as cyclooctene. Mixtures of olefins can be fed to the hydroformylation reaction zone, a typical mixture being propylene, isobutylene and butene-1. Such mixtures hydroformylate at production rates and at ratios of normal to isobutyraldehyde that are essentially equivalent to those achieved with propylene alone.

Recycled rhodium may be introduced as the carboxylate into the reaction zone along with solvent through liquid pressure pumping means. In the reaction zone the carboxylate interacts with the synthesis gas to form a complex, active catalyst species.

Solvents for use in hydroformylation processes are well known. Such solvents do not adversely affect the process and are inert with respect to the catalyst, olefin feed, synthesis gas and the hydroformylation products. Solvents of this nature include xylene and their substituted derivatives, pentanes, naphtha, kerosene, mineral oils, cyclohexane, cyclopentane, ethers, esters, etheresters, alcohols, acetals, ketones. Preferred solvents are benzene, toluene, ethanol, isopropanol, ethylene glycol monomethylether and ethylene glycol dimethylether, and most preferred solvents are 2,2,4-trimethyl-1,3-pentanediol monoisobutyrate (TMPDMI), and its isomers, and by-products such as alcohols, esters, acetals, and hydroxyaldehydes produced in the hydroformylation reaction that are retained as high-boiling liquids at the bottom of the distillation column. The solvent is preferably a high-boiling solvent and is introduced into the reaction unit along with the catalyst and allowed to recycle therewith. It should be particularly noted that when the effluent from the reaction unit (sometimes called simply reactor effluent) contains TMPDMI as a solvent, oxidation of the aldehyde product to acid is markedly retarded. From the standpoint of increased aldehyde yield and purity, this phenomenon is an unexpected adjunct of the present invention.

The present process can be carried out using small amounts of catalyst such as $1 \times 10^{-8}$ mol of rhodium (metal) per mole of olefin. However, such low catalyst concentrations are not desirable because the reaction rates are too low to be commercially attractive. The upper catalyst concentration appears to be dictated principally by the high cost of rhodium and the fact that no significant advantage is evident using catalysts containing above $1 \times 10^{-1}$ mol of rhodium per mole of olefin. A concentration of rhodium of from $1 \times 10^{-6}$ to $5 \times 10^{-2}$ mol per mole of olefin is preferred, and from $1 \times 10^{-4}$ to $1 \times 10^{-2}$ is most preferred.

The treatment with oxygen-containing gas, e.g., air, molecular oxygen or oxygen mixed with an inert gas, may be carried out at atmospheric conditions in simple equipment, such as a tank provided with means for continuous removal of catalyst. A wide range of temperatures may be used for the oxygen treatment such as from 25 to 80°C with a preferred range being from 50 to 60°C The reaction time required for efficient oxidation of the rhodium catalyst at 25 to 80°C can be up to 12 h or longer. Within the scope of this invention the preferred oxidation conditions are from 20 to 40 min at 50 to 60°C. The number of moles of oxygen per mole of rhodium for the oxidation is subject to wide variations, but it is preferred to employ a molar ratio of at least 1 (oxygen to rhodium) in excess of oxygen scavengers which may be present in the system.

The following example further illustrates the invention.

*Example:*

## A. *Preparation of Liquid Composition (Hydroformylation Reaction)*

A liquid composition for use in the air treatment and distillation procedures described hereinafter was prepared as follows:

Rhodium isobutyrate solution (10 ml, 0.1 mg Rh) was combined with 92 ml (83.2 g) of 2,2,4-trimethyl-1,3-pentanediol monoisobutyrate(TMPDMI) solvent in a 300-ml stainless steel autoclave equipped with a stirrer and automatic cooling. After chilling the autoclave in dry ice, propylene (40 g) was introduced. Synthesis gas (a mixture of hydrogen and carbon monoxide) was added to a pressure of 13,891 kPa (2,000 psig). The temperature was then raised to 150°C, and the pressure increased to 17,339 kPa (2,500 psig). This pressure was maintained for 30 min. The autoclave was cooled and vented. The weight gain and production rate of the butyraldehyde were determined. The liquid composition comprising catalyst solution and butyraldehyde product was pumped into a container which was stored under nitrogen at 5°C.

For the following Run 1 (air-treated), a part of the liquid compositions was air-oxidized for 30 min at 55°C to convert the catalyst to rhodium butyrate or isobutyrate. For the following Run 2 (untreated), part of the liquid composition was not oxidized and care was taken to avoid contact of the liquid composition with air.

## B. *Distillation Procedure*

This distillation procedure was used for both the air-treated and untreated liquid composition prepared in Procedure A.

For the distillation, the liquid composition was run into a conventional stainless steel distillation column. The distillation was initiated by starting the following: the base heater set at 120°C, the reboiler circulation pump, and the base take-off pump set at 600 ml/h in concert with a column feed pump which pumped liquid composition into the column at a rate which held a desired column level. Butyraldehyde was distilled and product containing catalyst was pumped into a tank while the column was brought to equilibrium. After the column reached equilibrium, an adequate amount of the equilibrium base product was collected for analysis in a second tank. The column was then shut down. Each column feed stream was analyzed by gas-liquid chromatography and the percent TMPDMI solvent, by weight, was determined. Gas-liquid chromatographic and atomic absorption spectroscopic analysis showed that no rhodium or TMPDMI solvent was distilled overhead from the column.

## C. *Determination of Rhodium Loss*

The loss of rhodium can be observed as the formation of rhodium deposits on the distillation equipment used in Procedure B. With the oxygen-treated liquid composition there is no evidence of such deposits. In contrast, upon distillation of the untreated liquid composition significant rhodium deposits are observed.

It is also possible to demonstrate the loss of rhodium in terms of catalytic activity. To illustrate, the hydroformylation reaction described in Procedure A followed by distillation as described in Procedure B was used to evaluate the catalytic activity of the following: (1) the air-treated liquid composition (before distillation), (2) the equilibrium base product obtained upon distillation of the air-treated liquid composition according to Procedure B, (3) the untreated liquid composition (before distillation), and (4) the equilibrium base product obtained upon distillation of the untreated liquid composition according to Procedure B.

In making the evaluations, samples of each of the liquid compositions (1) and (3) and their corresponding equilibrium base products (2) and (4) were weighed out to provide equal amounts of TMPDMI solvent by analysis. In theory, each of these samples would contain the same amount of rhodium if no rhodium was lost during distillation. The weight gains and production rates obtained with each sample were then determined and compared. The results were as follows:

*Distillation Results*

| | Aldehyde Production Rate $(kg/m^3/h)$ | |
|---|---|---|
| Run | Liquid Composition | Equilibrium Base Product |
| 1 (air-treated) | 716.1 | 761 |
| 2 (untreated) | 812.2 | 408.5 |

It is evident from the increased production of aldehyde in Run 1 that the equilibrium base product obtained from the air-treated liquid composition exhibited catalytic activity which was slightly increased with respect to that of the air-treated liquid composition. This illustrates that there was no loss of rhodium during distillation of the liquid composition treated according to this invention. In contrast, Run 2 shows that the equilibrium base product obtained from the untreated liquid composition upon distillation exhibited a substantial loss in catalytic activity (approximately 50%) in comparison to that of the untreated liquid composition. Also, when the distillation temperature used with the air-treated liquid composition is raised above 120°C, e.g., to 125 or 130°C, there is a substantial loss in catalytic activity and rhodium deposits form on the distillation equipment over the course of the distillation.

## Claims

1. A hydroformylation process in which the formation of rhodium deposits is inhibited, comprising (1) forming an aldehyde product in a liquid composition by hydroformylating an olefin in liquid solvent with hydrogen and carbon monoxide in the presence of a rhodium-containing catalyst which is capable of forming rhodium deposits on distillation apparatus during distillation of the liquid composition and (2) separating aldehyde product in the form of a gas from the liquid composition by distillation, characterized in that the liquid composition (a) is contacted with an oxygen-containing gas to thereby form a rhodium salt of a carboxylic acid and then (b) is subjected to the distillation at a temperature up to 120°C.

2. A process according to Claim 1, in which the olefin is an α- or internal olefin of 2 to 20 carbon atoms and it is hydroformylated at a temperature of from 20 to 300°C and a pressure of from 204 to 69,052 kPa.

3. Process according to Claim 1 or 2, in which liquid composition obtained from the distillation is returned to the hydroformylation reaction.

4. The process according to Claim 1, wherein the olefin is hydroformylated at a temperature of from 120 to 180°C and a pressure from 10,443 to 20,787 kPa.

5. The process according to any one of Claims 1 to 4, wherein the mole ratio of the hydrogen to the carbon monoxide is at least 0.5.

6. The process according to any one of Claims 1 to 5, wherein the olefin is ethylene, propylene, 1-butene, isobutylene, 2-methyl-1-butene, 1-pentene, 1-hexene, 1-heptene, 1-octene or a mixture thereof.

7. The process according to any one of Claims 1 to 6, wherein the solvent is benzene, toluene, ethanol, isopropanol, ethylene glycol monomethylether, ethylene glycol dimethylether, or 2,2,4-trimethyl-1,3-pentanediol monoisobutyrate.

8. A process according to Claim 7, wherein the solvent is 2,2,4-trimethyl-1,3-pentanediol monoisobutyrate.

9. A process according to any one of Claims 1 to 8, wherein the oxygen-containing gas is air, the mole ratio of oxygen to rhodium is at least 1 and the distillation temperature is less than 110°C.

10. The process according to any one of Claims 1 to 9, wherein the liquid composition is contacted with the oxygen-containing gas at a temperature of from 25 to 80°C.

## Patentansprüche

1. Hydroformylierungsverfahren, in dem der Bildung von Rhodiumniederschlägen entgegengewirkt wird, und bei dem 1) ein Aldehydprodukt in einer flüssigen Masse durch Hydroformylierung eines Olefins in einem flüssigen Lösungsmittel mit Wasserstoff und Kohlenmonoxid in Gegenwart eines Rhodium enthaltenden Katalysators, der Rhodiumniederschläge während der Destillation der flüssigen Masse auf der Destillationsapparatur zu bilden vermag, erzeugt wird, und bei dem 2) das Aldehydprodukt in Form eines Gases von der flüssigen Masse durch Destillation getrennt wird, dadurch gekennzeichnet, dass die flüssige Masse a) mit einem Sauerstoff enthaltenden Gas unter Bildung eines Rhodiumsalzes einer Carbonsäure in Kontakt gebracht wird und b) daraufhin der Destillation bei einer Temperatur von bis zu 120°C unterworfen wird.

2. Verfahren nach Anspruch 1, bei dem das Olefin ein α-Olefin oder ein inneres Olefin mit 2 bis 20 Kohlenstoffatomen ist und bei einer Temperatur von 20 bis 300°C und einem Druck von 204 bis 69052 kPa hydroformyliert wird.

3. Verfahren nach einem der Ansprüche 1 oder 2, bei dem bei der Destillation erhaltene flüssige Masse zur Hydroformylierungsreaktion zurückgeführt wird.

4. Verfahren nach Anspruch 1, bei dem das Olefin bei einer Temperatur von 120 bis 180°C und einem Druck von 10443 bis 20787 kPa hydroformyliert wird.

5. Verfahren nach einem der Ansprüche 1 bis 4, bei dem das Molverhältnis von Wasserstoff zu Kohlenmonoxid bei mindestens 0,5 liegt.

6. Verfahren nach einem der Ansprüche 1 bis 5, bei dem das Olefin aus Ethylen, Propylen, 1-Buten, Isobutylen, 2-Methyl-1-buten, 1-Penten, 1-Hexen, 1-Hepten, 1-Octen oder einer Mischung hiervon besteht.

7. Verfahren nach einem der Ansprüche 1 bis 6, bei dem das Lösungsmittel aus Benzol, Toluol, Ethanol, Isopropanol, Ethylenglykolmonomethylether, Ethylenglykoldimethylether oder 2,2,4-Trimethyl-1,3-pentandiolmonoisobutyrat besteht.

8. Verfahren nach Anspruch 7, bei dem das Lösungsmittel aus 2,2,4-Trimethyl-1,3-pentandiolmonoisobutyrat besteht.

9. Verfahren nach einem der Ansprüche 1 bis 8, bei dem das Sauerstoff enthaltende Gas aus Luft besteht, das Molverhältnis von Sauerstoff zu Rhodium bei mindestens 1 liegt und die Destillationstemperatur weniger als 110°C ist.

10. Verfahren nach einem der Ansprüche 1 bis 9, bei dem die flüssige Masse mit dem Sauerstoff enthaltenden Gas bei einer Temperatur von 25 bis 80°C in Kontakt gebracht wird.

## Revendications

1. Procédé d'hydroformylation dans lequel on inhibe la formation de dépôts de rhodium, comprenant 1) la formation d'un aldéhyde dans une composition liquide par hydroformylation d'une oléfine en milieu solvant liquide avec de l'hydrogène et du mono-oxyde de carbone en présence d'un catalyseur contenant du rhodium qui peut former des dépôts de rhodium sur l'appareil de distillation pendant la distillation de la composition liquide, et 2) la séparation de l'aldéhyde sous forme de gaz à partir de la composition liquide par distillation, caractérisé en ce que a) on met en contact la composition liquide avec un gaz contenant de l'oxygène, ce qui forme un sel de rhodium d'un acide carboxylique, et b) on soumet ensuite la composition liquide à la distillation jusqu'à une température de 120°C.

2. Procédé d'hydroformylation conforme à la revendication 1, caractérisé en ce que l'oléfine est une oléfine α ou interne de 2 à 20 atomes de carbone qui est hydroformylée à une température comprise entre 20 et 300°C à une pression comprise entre 204 et 69052 kPa.

3. Procédé d'hydroformylation conforme à l'une des revendications 1 ou 2, caractérisé en ce que la composition liquide obtenue par distillation est recyclée dans la réaction d'hydroformylation.

4. Procédé d'hydroformylation conforme à la revendication 1, caractérisé en ce que l'oléfine est hydroformylée à une température comprise entre 120 et 180°C et à une pression comprise entre 10443 et 20787 kPa.

5. Procédé d'hydroformylation conforme à l'une des revendications 1 à 4, caractérisé en ce que le rapport molaire de l'hydrogène au mono-oxyde de carbone est au moins égal à 0,5.

6. Procédé d'hydroformylation conforme à l'une des revendications 1 à 5, caractérisé en ce que l'oléfine est l'éthylène, le propylène, le 1-butène, l'isobutylène, le 2-méthyl-1-butène, le 1-pentène, le 1-hexène, le 1-heptène, le 1-octène ou leurs mélanges.

7. Procédé d'hydroformylation conforme à l'une des revendications 1 à 6, caractérisé en ce que le solvant est le benzène, le toluène, l'éthanol, l'isopropanol, le monométhyléther de l'éthylèneglycol, le diméthyléther de l'éthylèneglycol, ou le mono-isobutyrate du 2,2,4-triméthyl-1,3-penta-nediol.

8. Procédé conforme à la revendication 7, caractérisé en ce que le solvant est le mono-isobutyrate de 2,2,4-triméthyl-1,3-pentanediol.

9. Procédé d'hydroformylation conforme à l'une des revendications 1 à 8, caractérisé en ce que le gaz contenant l'oxygène est l'air, le rapport molaire de l'oxygène au rhodium est au moins égal à 1 et la température de distillation est inférieure à 110°C.

10. Procédé d'hydroformylation conforme à l'une des revendications 1 à 9, caractérisé en ce que l'on met la composition liquide en contact avec un gaz contenant de l'oxygène à une température comprise entre 25 et 80°C.